(19) Europäisches Patentamt / European Patent Office / Office européen des brevets

(11) **EP 2 974 651 A1**

(12) **EUROPEAN PATENT APPLICATION**

(43) Date of publication:
20.01.2016 Bulletin 2016/03

(51) Int Cl.:
*A61B 5/021* (2006.01)

(21) Application number: 14181718.9

(22) Date of filing: 21.08.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 14.07.2014 US 201414330133

(71) Applicant: MediaTek Inc.
Hsin-Chu 300 (TW)

(72) Inventor: Ku, Po-Wen
Jhubei City (TW)

(74) Representative: Habermann, Hruschka & Schnabel
Montgelasstrasse 2
81679 Munich (DE)

Remarks:
Amended claims in accordance with Rule 137(2) EPC.

(54) **Method for collecting personal health data and personal health device utilizing the same**

(57)     A personal health device includes multiple photoplethysmogram (PPG) sensors, multiple electrodes and a processing circuit. The PPG sensors are configured for sensing multiple PPG signals. The electrodes are configured for sensing multiple skin voltages. The processing circuit is coupled to the PPG sensors and the electrodes and configured for estimating an ankle brachial pressure index (ABI) according to the PPG signals and the skin voltages.

<u>100</u>

FIG. 1

**Description**

**BACKGROUND of the invention**

*Field of the Invention*

The invention relates to a personal health device convenient for personal health data collecting and monitoring.

*Description of the Related Art*

The effective functioning of many organs requires a sufficient blood supply. Control of blood circulation parameters is important both in cases of cardiovascular circulatory disturbances, and in prophylaxis. Arterial narrowings (occlusions) in arms and legs that hinder or even block blood supply constitute one of the most perilous diseases of blood circulation disturbances and may cause serious organic changes.

Several methods of diagnostics of arterial occlusions are known, e.g. arterial oscillography, local registration of blood pressure and determination of the ankle/arm index.

Disadvantages of the known methods include the inconvenience of their use (constrictors, pressure cuffs with compression and decompression equipment, etc.), complicated procedures (several manipulations in a specific order), relatively long duration (typically about 30 minutes) and the difficulty of applying the methods outside of hospitals (e.g. in the field or under domestic conditions), because of the massive stationary equipment required.

**Brief Summary of the Invention**

The personal health device, computing device, method for collecting personal health data and computer readable storage medium are provided. An exemplary embodiment of a personal health device comprises a plurality of photoplethysmogram (PPG) sensors, a plurality of electrodes and a processing circuit. The PPG sensors are configured for sensing a plurality of PPG signals. The electrodes are configured for sensing a plurality of skin voltages. The processing circuit is coupled to the plurality of PPG sensors and the plurality of electrodes and configured for estimating an ankle brachial pressure index (ABI) according to the plurality of PPG signals and the plurality of skin voltages.

An exemplary embodiment of a computing device comprises a processing circuit. The processing circuit comprises a processor configured for estimating an ankle brachial pressure index (ABI) according to a plurality of photoplethysmogram (PPG) signals and at least one electrocardiography (ECG) lead signal.

An exemplary embodiment of a method for collecting personal health data comprises: sensing a plurality of photoplethysmogram (PPG) signals of a user; sensing a plurality of skin voltages of the user; estimating an ankle brachial pressure index (ABI) according to the plurality of PPG signals and the plurality of skin voltages.

An exemplary embodiment of a computer readable storage medium having stored therein instructions, which when executed by a device, cause the device to estimate an ankle brachial pressure index (ABI) according to a plurality of PPG signals and at least one ECG lead signal.

A detailed description is given in the following embodiments with reference to the accompanying drawings.

**BRIEF DESCRIPTION OF DRAWINGS**

The invention can be more fully understood by reading the subsequent detailed description and examples with references made to the accompanying drawings, wherein:

FIG. 1 is an exemplary block diagram of a personal health device according to an embodiment of the invention;
FIG. 2 is a diagram showing a conventional way of measuring the ABI;
FIG. 3 shows an exemplary appearance of a personal health device according to an embodiment of the invention;
FIG. 4 shows a perspective drawing of an exemplary sensor housing body according to an embodiment of the invention;
FIG. 5 shows an exemplary PPG signal waveform according to an embodiment of the invention;
FIG. 6 shows an exemplary ECG lead signal waveform according to an embodiment of the invention;
FIG. 7 is a schematic diagram showing a concept of sensing the skin voltages in different body parts of a user to obtain different ECG lead signals according to an embodiment of the invention;
FIG. 8 is an exemplary diagram showing the PWTT between the R-peak of the ECG lead signal and a systolic peak of the PPG signal according to an embodiment of the invention;
FIG. 9 is an exemplary diagram showing three different PATs measured between the R-peak of the ECG lead signal and the PPG signal according to an embodiment of the invention;

FIG. 10 is a schematic diagram showing a concept of estimating the ABI by using the personal health device as illustrated above according to an embodiment of the invention;

FIG. 11 is another exemplary block diagram of a personal health device according to another embodiment of the invention; and

FIG. 12 is a flow chart showing a method for collecting personal health data according to an embodiment of the invention.

**Detailed Description of the Invention**

The following description is of the best-contemplated mode of carrying out the invention. This description is made for the purpose of illustrating the general principles of the invention and should not be taken in a limiting sense. The scope of the invention is best determined by reference to the appended claims.

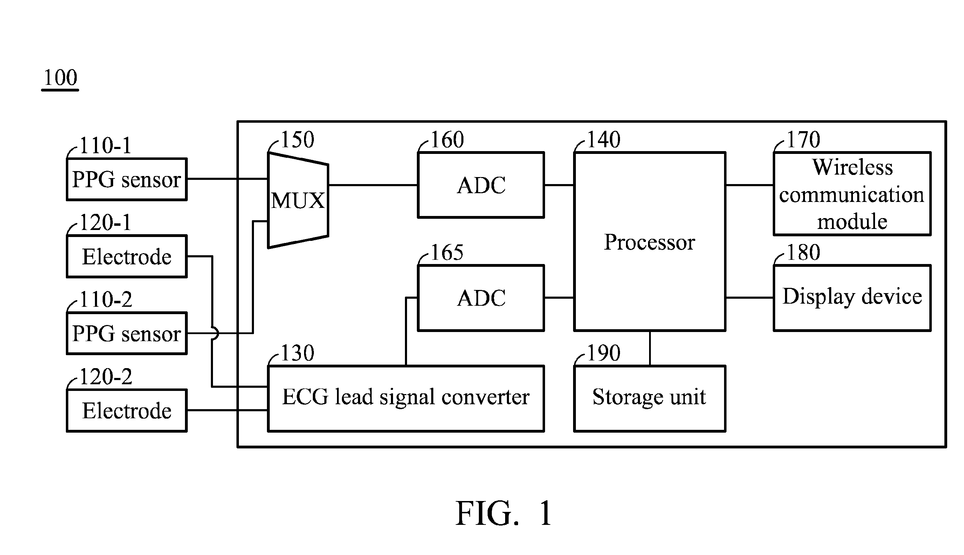

FIG. 1 is an exemplary block diagram of a personal health device according to an embodiment of the invention. According to an embodiment of the invention, the personal health device 100 may comprise at least a plurality of photoplethysmogram (PPG) sensors, such as the PPG sensors 110-1 and 110-2, a plurality of electrodes, such as the electrodes 120-1 and 120-2, an ECG lead signal converter 130 and a processor 140.

The PPG sensors 110-1 and 110-2 are configured for sensing a plurality of PPG signals. The PPG sensors are used to estimate the perfusion of the blood, i.e. the volume change of the blood flow in the dermis and subcutaneous tissue of the skin. One application of the PPG sensors is used to estimate the degree of oxygenation in arterial blood. For example, red and infrared light is emitted towards a body part. The infrared light is more strongly absorbed by oxygenated blood than by non-oxygenated blood; red light is more strongly absorbed by non-oxygenated blood than by oxygenated blood. The change in the infrared absorption during systole is a measure of the amount of oxygenated blood. The level of red light absorption between systoles is a measure of the total amount of blood being illuminated and is used for calibration.

The electrodes 120-1 and 120-2 are configured for sensing a plurality of skin voltages. The ECG lead signal converter 130 is coupled to the plurality of electrodes 120-1 and 120-2 for receiving the plurality of skin voltages and generates at least one ECG lead signal according to the plurality of skin voltages.

The personal health device 100 may further comprise a multiplexer (MUX) 150 and analog-to-digital converters (ADC) 160 and 165. The MUX 150 is coupled to the PPG sensors 110-1 and 110-2 for multiplexing the PPG signals to the ADC 160. The ADC 160 is configured for performing analog-to-digital conversion of the PPG signals and provides the converted PPG signals to the processor 140. The processor 140 may be a general purpose processor or a digital signal processor (DSP) which receives a specific instruction set to execute certain tasks. However, it is feasible to use dedicate hardware or fixed function circuits to replace the processor 140. The ADC 165 is configured for performing analog-to-digital conversion of the ECG lead signal and provides the converted ECG lead signal to the processor 140. Note that, in some embodiments of the invention, the ADC 160 and ADC 165 may be combined as one ADC with an additional multiplexer utilized for multiplexing the PPG signals and the ECG lead signal to the combined ADC. In some other embodiments of the invention, the PPG sensor 110-1 and PPG sensor 110-2 may each be coupled to an ADC, respectively; and the MUX 150 may be removed. Therefore, FIG. 1 is merely one of a plurality of possible embodiments of the proposed personal health device, and the invention should not be limited thereto.

In practice, the ECG lead signal converter 130, the MUX 150, the ADC 160, the ADC 165 and the processor 140 may be implemented within an application specific integrated circuit (ASIC). These blocks may be altogether referred to as a processing circuit. The processing circuit is configured for estimating an ankle brachial pressure index (ABI) according to the plurality of PPG signals and the plurality of skin voltages. The ABI is the ratio of the blood pressure in the lower legs to the blood pressure in the arms. Compared to the blood pressure of the arm, lower blood pressure in the leg is an indication of blocked arteries (Peripheral Vascular Disease (PVD) or Peripheral Arterial Disease (PAD)). In general, the ABI is calculated by dividing the systolic blood pressure at the ankle by the systolic blood pressure in the arm. As the ABI decreases from 1, it suggests the PAD is getting more severe. As for the blood pressures of the arm and the leg, they may be derived according to the PPG signals and the ECG lead signal, which will be explained with more detail later.

Hence, according to an embodiment of the invention, the personal health device 100 may comprise a plurality of PPG sensors, a plurality of electrodes and a processing circuit. The plurality of PPG sensors are configured for sensing a plurality of PPG signals. The plurality of electrodes is configured for sensing a plurality of skin voltages. The processing circuit is coupled to the plurality of PPG sensors and the plurality of electrodes; and the processing circuit is configured for estimating an ABI according to the plurality of PPG signals and the plurality of skin voltages. Specifically, the processing circuit may comprise an electrocardiography (EGC) lead signal converter, a blood pressure calculator and an ABI generator. The ECG lead signal converter is configured for providing at least one ECG lead signal according to the plurality of skin voltages. The blood pressure calculator is configured for estimating a plurality of blood pressures according to the plurality of PPG signals and the at least one ECG lead signal. The ABI generator is configured for calculating the ABI according to the plurality of blood pressures.

To be reminded, the blood pressure calculator and the ABI generator may be replaced by a processor, which executes instructions for estimating a plurality of blood pressures according to the plurality of PPG signals and the at least one ECG lead signal; and calculating the ABI according to the plurality of blood pressures. The instructions executed by the processor may be provided in the form of an application program. When a user wants to know the ABI, the application program may be downloaded from a computer readable storage medium such as an internet disk drive, a cloud storage or an optical disk to the processor. When the application program is run on the processor, the processor executes the instructions to estimate the ABI according to the plurality of PPG signals and the at least one ECG lead signal.

According to an embodiment of the invention, the plurality of PPG signals may comprise a first PPG signal and a second PPG signal, the at least one ECG lead signal may comprise a first ECG lead signal, the plurality of blood pressures may comprise a first blood pressure and a second blood pressure. The first blood pressure may be estimated according to the first PPG signal and the first ECG lead signal, and the second blood pressure may be estimated according to the second PPG signal and the first ECG lead signal. The ABI is estimated according to the first blood pressure and the second blood pressure.

According to a preferred embodiment of the invention, the plurality of PPG signals and the plurality of skin voltages are preferably sensed substantially simultaneously. However, the invention should not be limited thereto.

According to an embodiment of the invention, the personal health device 100 may further comprise a wireless communication module 170, a display device 180 and a storage unit 190. The wireless communication module 170 is configured for transmitting information regarding the plurality of PPG signals, the at least one ECG lead signal, the plurality of blood pressures, or the ABI to an electronic device. The electronic device may be a mobile phone, a computer, a cloud server, or others. The display device 180 is configured for providing a user interface to the user using the personal health device 100 and displaying estimated ABI. The storage unit 190 is configured for storing a plurality of ABI records estimated at different times. The processor 140 may monitor changes of the plurality of ABI records of each user for detecting the beginning or degeneration of the PVD or PAD. In the embodiments of the invention, the personal health device 100 may be powered by battery power or utility power, and the power supply device is not shown in FIG. 1 for brevity.

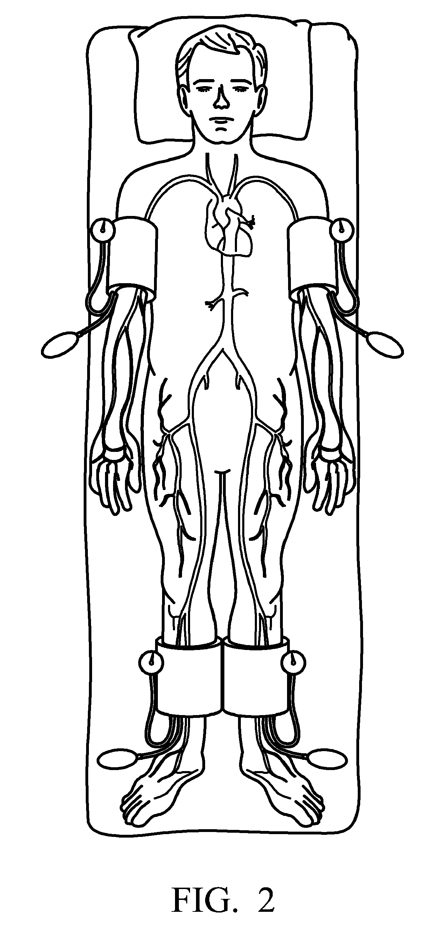

FIG. 2 is a diagram showing a conventional way of measuring the ABI. As shown in FIG. 2, the conventional way of measuring the ABI uses the blood pressure cuffs, which is inconvenient and difficult to apply outside of a hospital. Compared to the conventional way of measuring the ABI, the proposed methods and personal health devices are more convenient for the user and can be applied anywhere outside hospital, such as allowing the user to estimate the ABI at home and keep monitoring the ABI for a period of time. The proposed methods and personal health devices are discussed further in the following paragraphs.

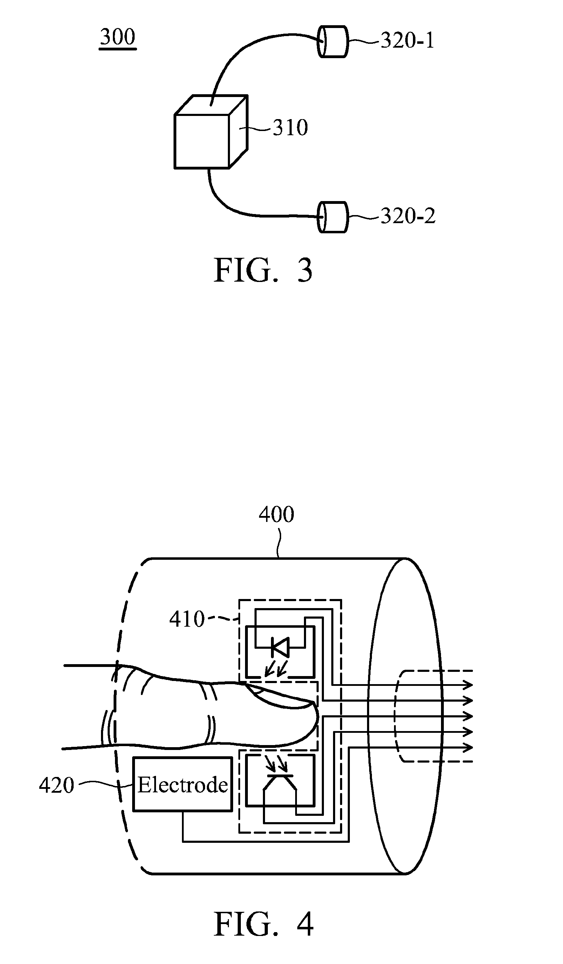

FIG. 3 shows an exemplary appearance of a personal health device according to an embodiment of the invention. The personal health device 300 may comprise a main housing body 310 and a plurality of sensor housing bodies, such as the sensor housing bodies 320-1 and 320-2. In addition, the sensor housing bodies are wired connected to the main housing body 310. Note that the personal health device may comprise more than two sensor housing bodies, and the invention should not be limited thereto.

Referring to FIG. 1 and FIG. 3, according to an embodiment of the invention, one of the plurality of PPG sensors and one of the plurality of electrodes may be housed in a first sensor housing body, such as the sensor housing body 320-1, and another of the plurality of PPG sensors and another of the plurality of electrodes may be housed in a second sensor housing body, such as the sensor housing body 320-2. The remaining components in the personal health device 100 may be housed in the main housing body 310. For instance, the PPG sensor 110-1 and the electrode 120-1 are housed in the sensor housing body 320-1; the PPG sensor 110-2 and the electrode 120-2 are housed in the sensor housing body 320-2; and the ECG lead signal converter 130, the MUX 150, the ADC 160, the ADC 165, the processor 140, the wireless communication module 170, the display device 180 and the storage unit 190 are housed in the main housing body 310.

According to an embodiment of the invention, one of the plurality of PPG signals and one of the plurality of skin voltages are sensed by placing the first sensor housing body on the upper limb of the user, and another of the plurality of PPG signals and another of the plurality of skin voltages are sensed by placing the second sensor housing body on a lower limb of the user. For instance, the sensor housing body 320-1 is placed on the right arm of the user and the sensor housing body 320-2 is placed on the left leg of the user.

FIG. 4 shows a perspective drawing of an exemplary sensor housing body according to an embodiment of the invention. In this example, the sensor housing body 400 is placed on a finger of user for sensing at least one PPG signal via the PPG sensor 410 and sensing at least one skin voltage via the electrode 420. The PPG sensor 410 may comprise an infrared transmitter LED for transmitting light and a photo sensor for sensing light transmitted through the finger of the user.

Note that in some embodiments of the invention, the PPG sensors and the electrodes may also be disposed in different sensor housing bodies. For example, the personal health device 300 may comprise more than two sensor housing bodies. One of the PPG sensors may be housed in a first sensor housing body, and another of the PPG sensors may be housed in a second sensor housing body. One of the electrodes may be housed in a third sensor housing body, and another of the plurality of electrodes may be housed in a fourth sensor housing body. Therefore, FIG. 4 only shows one of a plurality of possible implementations of the sensor housing body, and the invention should not be limited to thereto.

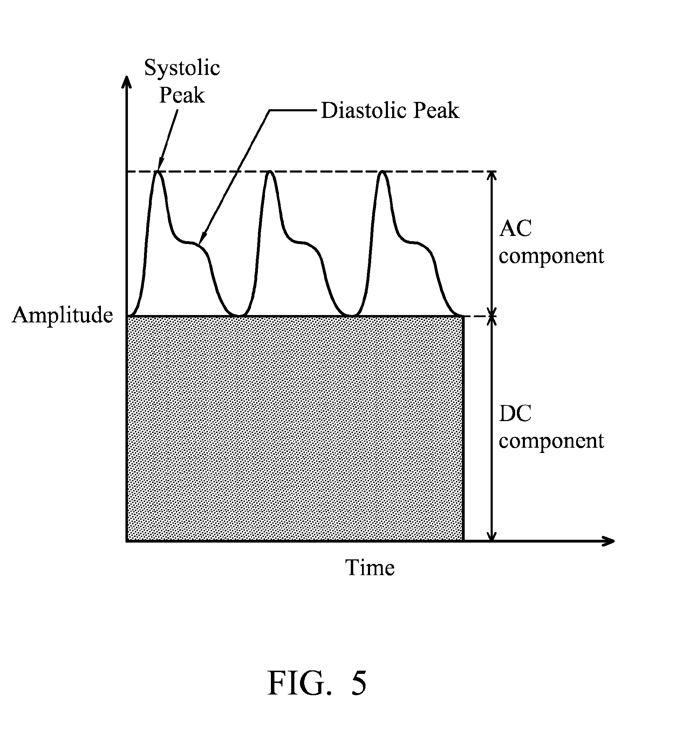

FIG. 5 shows an exemplary PPG signal waveform according to an embodiment of the invention. As shown in FIG. 5, the PPG signal comprises an AC component and a DC component. The systolic peak and diastolic peak may be obtained from the AC component. Since the PPG signals are utilized for estimating the ABI, the PPG signals preferably comprise at least a first PPG signal obtained by sensing an upper limb of a user and a second PPG signal obtained by sensing a lower limb of the user. Note that, in some embodiments of the invention, the ABI may also be estimated according to more than two PPG signals, and the invention should not be limited thereto.

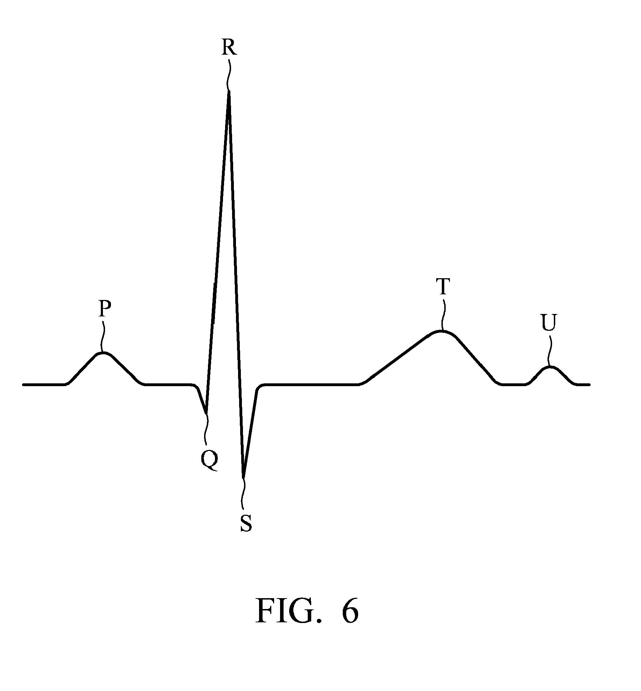

FIG. 6 shows an exemplary ECG lead signal waveform according to an embodiment of the invention. According to an embodiment of the invention, the ECG lead signal converter may first amplify the received skin voltages via an instrumentation amplifier (IA), which may be a differential amplifier, and then filter the amplified signal to obtain the ECG lead signal. According to an embodiment of the invention, as long as the skin voltages are obtained from two different body parts of a user and a line between the two body parts across the heart of the user, a corresponding ECG lead signal can be obtained. Therefore, in the embodiments of the invention, the skin voltages can be obtained by sensing any two body parts as long as a line between the two body parts across the heart of the user.

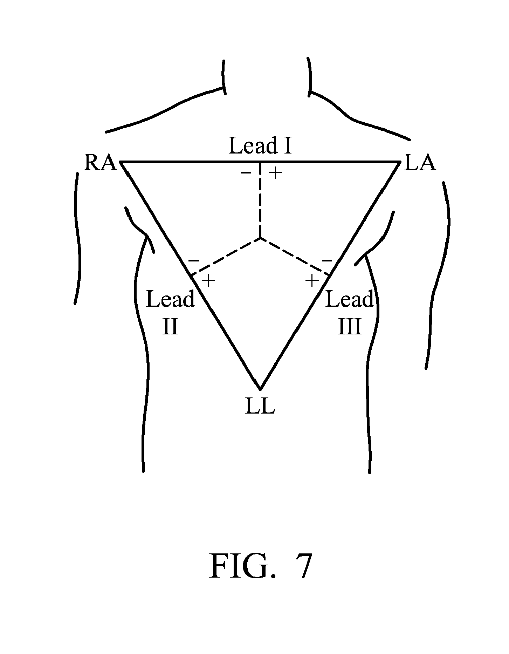

FIG. 7 is a schematic diagram showing a concept of sensing the skin voltages in different body parts of a user to obtain different ECG lead signals according to an embodiment of the invention. As shown in FIG. 7, when sensing the skin voltages in different body parts of the user, different ECG lead signals, such as the Lead I, Lead II and Lead III can be obtained. Therefore, although in the embodiments, the ECG lead signal is utilized for estimating the ABI, the skin voltages utilized for generating the ECG lead signal are not limited to be obtained by sensing the arm and leg. In addition, the skin voltages utilized for generating the ECG lead signal are not limited to be obtained by sensing the arm and leg on the same side. For example, the skin voltages obtained from the left arm and the right leg and the skin voltages obtained from the right arm and the left leg are both applicable.

According to an embodiment of the invention, a blood pressure may be estimated according to a Pulse Wave Transit Time (PWTT) between a peak of the ECG lead signal and a peak of the PPG signal, or a Pulse Arrival Time (PAT) of the PPG signal.

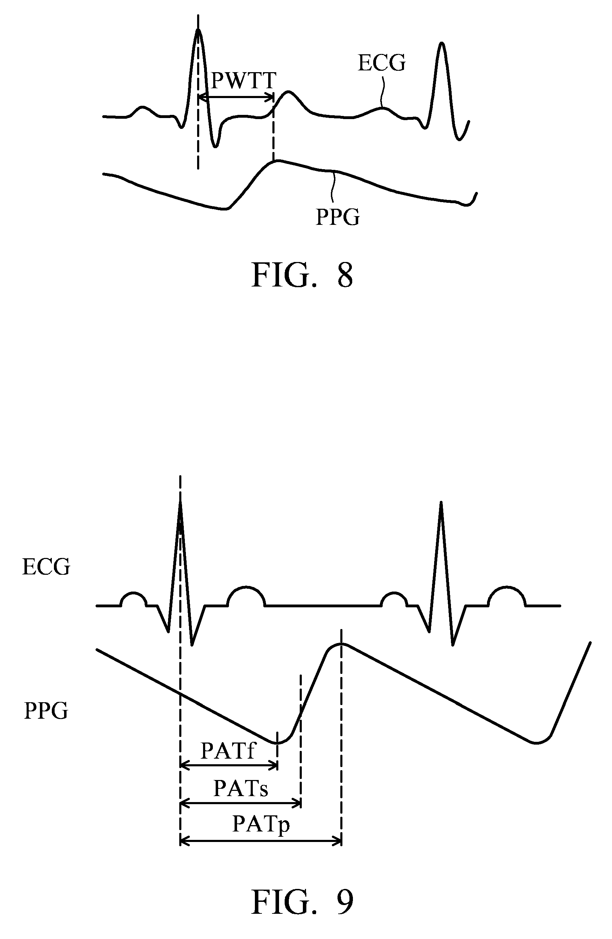

FIG. 8 is an exemplary diagram showing the PWTT between the R-peak of the ECG lead signal and a systolic peak of the PPG signal according to an embodiment of the invention. The PWTT is the time interval for the arterial pulse pressure wave to travel from the aortic valve to a peripheral site. An acute rise in blood pressure causes vascular tone to increase and the arterial wall become stiffer, causing the PWTT to shorten. Therefore, the PWTT varies inversely with blood pressure changes. To be more specific, the shorter PWTT implies a higher blood pressure, and vice versa.

FIG. 9 is an exemplary diagram showing three different PATs measured between the R-peak of the ECG lead signal and the PPG signal according to an embodiment of the invention. The PATf is the time interval between the R-peak of the ECG lead signal and a foot point of the PPG signal. The PATs is the time interval between the R-peak of the ECG lead signal and a slope peak of the PPG signal. The PATp is the time interval between the R-peak of the ECG lead signal and a peak of the PPG signal. The PAT varies inversely with blood pressure changes.

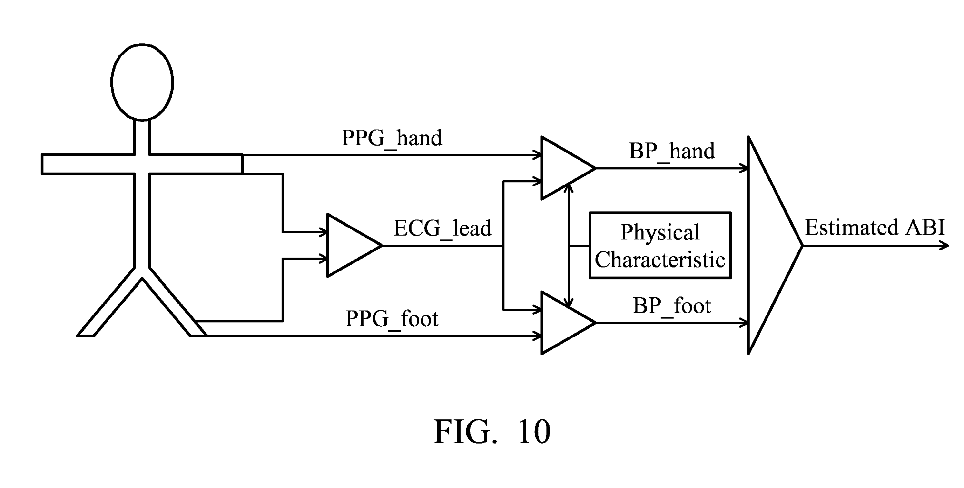

FIG. 10 is a schematic diagram showing a concept of estimating the ABI by using the personal health device as illustrated above according to an embodiment of the invention. Referring to FIG. 3 and FIG. 10, a first PPG signal PPG_hand and a second PPG signal PPG_foot may be respectively obtained by placing the sensor housing body 320-1 of the personal health device 300 on a finger of the user and by placing the sensor housing body 320-2 of the personal health device 300 on a toe of the user. The ECG lead signal ECG_lead is obtained according to at least two sensed skin voltages, one provided by placing the sensor housing body 320-1 on the finger of the user and another provided by placing the sensor housing body 320-2 on the toe of the user. A first blood pressure BP_hand is then obtained according to the first PPG signal PPG_hand and the ECG lead signal ECG_lead. A second blood pressure BP_foot is then obtained according to the second PPG signal PPG_ foot and the ECG lead signal ECG_lead.

According to an embodiment of the invention, the blood pressure may be estimated based on one or more models or algorithms. A simple model for estimating the blood pressure can be derived as below:

$$SBP = a * PWTT + b \qquad \text{Eq. (1)}$$

where SBP represents the systolic blood pressure and a and b are predetermined parameters. Note that the Eq. (1) is merely one of a plurality of possible implementations of the model, and the invention should not be limited thereto. For example, the blood pressure may also be derived according to more complicated models or algorithms, and may also be estimated further according to physical characteristics of the user, such as the height, weight, age, rhythm of the heart, or others.

After obtaining the blood pressures BP_hand and BP_foot, the ABI may be estimated according to the blood pressures BP_hand and BP_foot. Note that in some embodiments of the invention, the ABI may also be estimated further according to physical characteristics of the user, and the invention should not be limited thereto. Note further that the finger and toe utilized for sensing the PPG signals and the skin voltages as shown in FIG. 10 are merely one example of a plurality of possible implementations. As will be readily appreciated by one of ordinary skill in the art, the sensor housing bodies of the personal health device may also be placed on any body parts of the upper limb and lower limb of the user, and the invention should not be limited thereto.

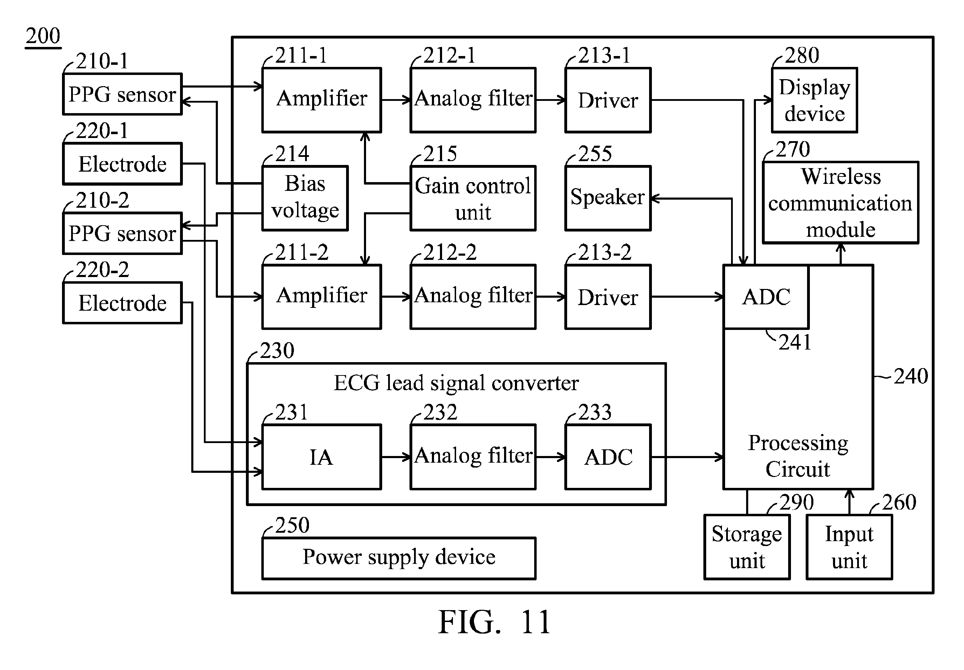

FIG. 11 is another exemplary block diagram of a personal health device according to another embodiment of the invention. The personal health device 200 shown in FIG. 11 may comprise more elements than the personal health device 100 shown in FIG. 1. However, it should be noted that FIG. 1 and FIG. 11 are merely two of a plurality of possible implementations of the personal health device, and the invention should not be limited thereto.

The personal health device 200 may comprise at least a plurality of photoplethysmogram (PPG) sensors, such as the PPG sensors 210-1 and 210-2, a plurality of electrodes, such as the electrodes 220-1 and 220-2, an ECG lead signal converter 230, a processing circuit 240, a power supply device 250, a speaker 255, an input unit 260, a wireless communication module 270, a display device 280 and a storage unit 290. Regarding the descriptions of the PPG sensors 210-1 and 210-2, the electrodes 220-1 and 220-2, the ECG lead signal converter 230, the processing circuit 240, the wireless communication module 270, the display device 280 and the storage unit 290, reference may be made to the corresponding elements of FIG. 1, and are omitted here for brevity.

The power supply device 250 is configured for providing utility power or battery power. The speaker 255 is configured for playing sounds to notify the user operating the personal health device 200. The input unit 260 is configured for facilitating the user to input commands or physical characteristics. According to an embodiment of the invention, the personal health device 200 may further comprise amplifiers 211-1 and 211-2 configured for amplifying the PGG signals received from the PPG sensors 210-1 and 210-2, analog filters 212-1 and 212-2 configured for filtering the amplified PGG signals and drivers 213-1 and 213-2 configured for further driving the filtered PGG signals before being provided to the processing circuit 240. The processing circuit 240 may comprise an ADC 241 integrated therein and configured for performing analog to digital conversion.

Note that the amplifiers 211-1 and 211-2 may be integrated into one device, and as well as the analog filters 212-1 and 212-2 and the drivers 213-1 and 213-2, and a multiplexer may be added for multiplexing the PPG signals as shown in FIG. 1.

The personal health device 200 may further comprise a bias voltage 214 configured for providing the bias voltage to the transmitter LED in the PPG sensors 210-1 and 210-2, and a gain control unit 215 configured for controlling the gain value of the amplifiers 211-1 and 211-2. The ECG lead signal converter 230 may comprise an instrumentation amplifier (IA) 231 configured for amplifying the skin voltages received from the electrodes 220-1 and 220-2, an analog filter 232 configured for filtering the amplified skin voltages and an ADC 233 configured for performing analog to digital conversion.

Note that in some embodiments of the invention, the personal health device 100/200/300 may comprise more than two PPG sensors and two electrodes, and the blood pressure may also be derived according to the models or algorithms that are more complicated and different from the model shown in Eq. (1). For example, a blood pressure may be derived according to more than one PPG signal and more than one ECG lead signal. Therefore, the embodiments as illustrated above are merely some of a plurality of possible implementations, and the invention should not be limited thereto.

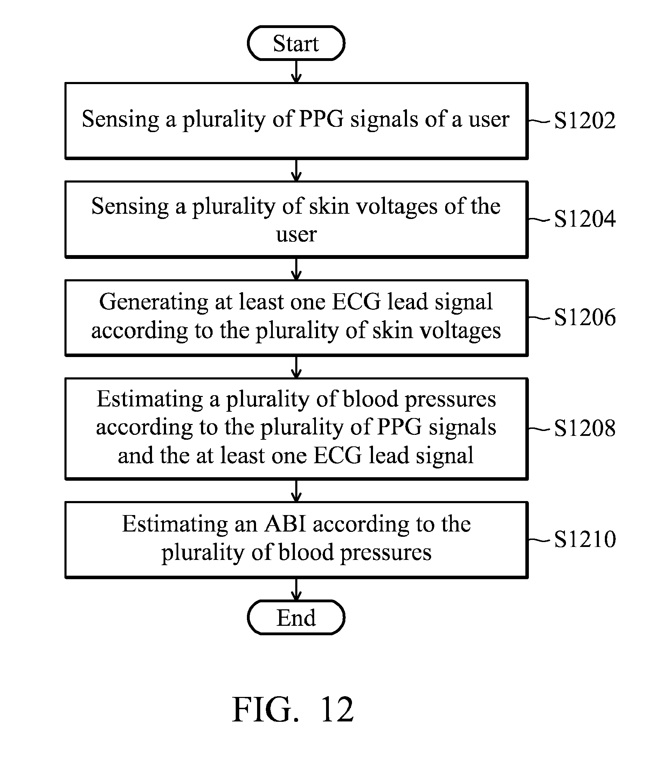

FIG. 12 is a flow chart showing a method for collecting personal health data according to an embodiment of the invention. A plurality of PPG signals of a user are sensed via a plurality of PPG sensors comprised in the personal health device (Step S1202) and a plurality of skin voltages of the user are sensed via a plurality of electrodes comprised in the personal health device (Step S1204). Next, at least one ECG lead signal is generated according to the plurality of skin voltages (Step S1206). Next, a plurality of blood pressures are estimated according to the plurality of PPG signals and the at least one ECG lead signal (Step S1208). An ABI is estimated according to the plurality of blood pressures (Step S1210). Note that, in practice, some or all of the five steps listed above may be carried out concurrently.

As discussed above, comparing to the conventional way of measuring the ABI, the proposed methods and personal health devices are more convenient for the user and can be applied anywhere outside of a hospital, such as estimating the ABI at home and keep monitoring the ABI records estimated at different times for a period of time. In this manner, the beginning or degeneration of the PVD or PAD may be detected as early as possible. In addition, since ABI estimated by the proposed personal health devices is a ratio of estimated blood pressures, even if the estimated blood pressures may not be very accurate, the ratio of estimated blood pressures and the change of the ABI records estimated at different times are still very meaningful for the user to monitor his/her personal health.

While the invention has been described by way of example and in terms of preferred embodiment, it is to be understood that the invention is not limited thereto. Those who are skilled in this technology can still make various alterations and modifications without departing from the scope and spirit of this invention. Therefore, the scope of the present invention shall be defined and protected by the following claims and their equivalents.

**Claims**

1. A personal health device, comprising:

   a plurality of photoplethysmogram (PPG) sensors, configured for sensing a plurality of PPG signals;
   a plurality of electrodes, configured for sensing a plurality of skin voltages; and
   a processing circuit, coupled to the plurality of PPG sensors and the plurality of electrodes, configured for estimating an ankle brachial pressure index (ABI) according to the plurality of PPG signals and the plurality of skin voltages.

2. The personal health device as claimed in claim 1, further comprising a first sensor housing body and a second sensor housing body, wherein one of the plurality of PPG sensors and one of the plurality of electrodes are housed in the first sensor housing body, and another of the plurality of PPG sensors and another of the plurality of electrodes are housed in the second sensor housing body.

3. The personal health device as claimed in claim 2, further comprising a main housing body, wherein the processing circuit is housed in the main housing body, and wherein the first sensor housing body and the second sensor housing body are wired connected to the main housing body.

4. The personal health device as claimed in claim 2, wherein one of the plurality of PPG signals and one of the plurality of skin voltages are sensed by placing the first sensor housing body on an upper limb of a user, and another of the plurality of PPG signals and another of the plurality of skin voltages are sensed by placing the second sensor housing body on a lower limb of the user.

5. A computing device, comprising:

   a processing circuit, comprising:

      a processor, configured for estimating an ankle brachial pressure index (ABI) according to a plurality of photoplethysmogram (PPG) signals and at least one electrocardiography (ECG) lead signal.

6. The computing device as claimed in claim 5, wherein the processor executes instructions for:

   estimating a plurality of blood pressures according to the plurality of PPG signals and the at least one ECG lead signal; and
   calculating the ABI according to the plurality of blood pressures.

7. The computing device as claimed in claim 6, wherein the plurality of PPG signals comprise a first PPG signal and a second PPG signal, and the at least one ECG lead signal comprises a first ECG lead signal, and the plurality of blood pressures comprise a first blood pressure and a second blood pressure, wherein the first blood pressure is estimated according to the first PPG signal and the first ECG lead signal, and the second blood pressure is estimated according to the second PPG signal and the first ECG lead signal, and the ABI is calculated according to the first blood pressure and the second blood pressure.

8. The computing device as claimed in claim 5, further comprising a storage unit, configured for storing a plurality of ABI records estimated at different times.

9. A method for collecting personal health data, comprising:

   sensing a plurality of photoplethysmogram (PPG) signals of a user;
   sensing a plurality of skin voltages of the user;
   estimating an ankle brachial pressure index (ABI) according to the plurality of PPG signals and the plurality of skin voltages.

**10.** The method as claimed in claim 9, wherein the step of estimating the ABI comprises:

providing at least one ECG lead signal according to the plurality of skin voltages;
estimating a plurality of blood pressures according to the plurality of PPG signals and the at least one ECG lead signal; and
calculating the ABI according to the plurality of blood pressures.

**11.** The method as claimed in claim 10, wherein the plurality of PPG signals comprise a first PPG signal and a second PPG signal, and the at least one ECG lead signal comprises a first ECG lead signal, and the plurality of blood pressures comprise a first blood pressure and a second blood pressure, wherein the first blood pressure is estimated according to the first PPG signal and the first ECG lead signal, and the second blood pressure is estimated according to the second PPG signal and the first ECG lead signal, and the ABI is calculated according to the first blood pressure and the second blood pressure.

**12.** The method as claimed in claim 9, wherein the plurality of PPG signals and the plurality of skin voltages are sensed substantially simultaneously.

**13.** A computer readable storage medium having stored therein instructions, which when executed by a device, cause the device to:

estimate an ankle brachial pressure index (ABI) according to a plurality of PPG signals and at least one ECG lead signal.

**14.** The computer readable storage medium as claimed in claim 13, wherein estimating the ABI comprises:

estimating a plurality of blood pressures according to the plurality of PPG signals and the at least one ECG lead signal; and
calculating the ABI according to the plurality of blood pressures.

**15.** The computer readable storage medium as claimed in claim 14, wherein the plurality of PPG signals comprise a first PPG signal and a second PPG signal, and the at least one ECG lead signal comprises a first ECG lead signal, and the plurality of blood pressures comprise a first blood pressure and a second blood pressure, wherein the first blood pressure is estimated according to the first PPG signal and the first ECG lead signal, and the second blood pressure is estimated according to the second PPG signal and the first ECG lead signal, and the ABI is calculated according to the first blood pressure and the second blood pressure.

**Amended claims in accordance with Rule 137(2) EPC.**

**1.** A personal health device (100), comprising:

a plurality of photoplethysmogram (PPG) sensors (110-1, 110-2), configured for sensing a plurality of PPG signals;
a plurality of electrodes (120-1, 120-2), configured for sensing a plurality of skin voltages; and
a processing circuit, coupled to the plurality of PPG sensors (110-1, 110-2) and the plurality of electrodes, (120-1, 120-2) configured for
estimating an ankle brachial pressure index (ABI) according to the plurality of PPG signals and the plurality of skin voltages,

**characterized by**
the personal health device (100), further comprising a first sensor housing body (320-1) and a second sensor housing body (320-2), wherein one of the plurality of PPG sensors (110-1, 110-2) and one of the plurality of electrodes (120-1, 120-2) are housed in the first sensor housing body (320-1), and another of the plurality of PPG sensors (110-1, 110-2) and another of the plurality of electrodes are (120-1, 120-2) are housed in the second sensor housing body (320-2).

**2.** The personal health device (100) as claimed in claim 1, further comprising a main housing body, wherein the processing circuit is housed in the main housing body, and wherein the first sensor housing body (320-1), and and wherein the first sensor housing body (320-1), and the second sensor housing body (320-2), are wired connected to the main housing body.

3. The personal health device (100) as claimed in claim 1, wherein one of the plurality of PPG signals and one of the plurality of skin voltages are sensed by placing the first sensor housing body (320-1) on an upper limb of a user, and another of the plurality of PPG signals and another of the plurality of skin voltages are sensed by placing the second sensor housing body (320-2) on a lower limb of the user.

4. The device (100) as claimed in any one of the preceding claims, wherein the processing circuit executes instructions for:

estimating a plurality of blood pressures according to the plurality of PPG signals and the at least one ECG lead signal; and calculating the ABI according to the plurality of blood pressures.

5. The computing device as claimed in claim 4, wherein the plurality of PPG signals comprise a first PPG signal and a second PPG signal, and the at least one ECG lead signal comprises a first ECG lead signal, and the plurality of blood pressures comprise a first blood pressure and a second blood pressure, wherein the first blood pressure is estimated according to the first PPG signal and the first ECG lead signal, and the second blood pressure is estimated according to the second PPG signal and the first ECG lead signal, and the ABI is calculated according to the first blood pressure and the second blood pressure.

6. The device as claimed in any one of the preceding claims, further comprising a storage unit (190), configured for storing a plurality of ABI records estimated at different times.

7. A method for collecting personal health data, comprising:

sensing a plurality of photoplethysmogram (PPG) signals of a user (S1202);
sensing a plurality of skin voltages of the user (S1204);
estimating an ankle brachial pressure index (ABI) according to the plurality of PPG signals and the plurality of skin voltages,

wherein said plurality of PPG signals being provided by a plurality of sensors (110-1, 110-2), and said plurality of skin voltages being provided by a plurality of electrodes (120-1, 120-2),
**characterized by**
one of the plurality of PPG sensors (110-1, 110-2) and one of the plurality of electrodes (120-1, 120-2) are housed in a first sensor housing body (320-1), and another one of the plurality of PPG sensors (110-1, 110-2) and another one of the plurality of electrodes (120-1, 120-2) are housed in the second sensor housing body (320-2).

8. The method as claimed in claim 7, wherein the step of estimating the ABI comprises:

providing at least one ECG lead signal according to the plurality of skin voltages;
estimating a plurality of blood pressures according to the plurality of PPG signals and the at least one ECG lead signal (S1208); and
calculating the ABI according to the plurality of blood pressures (S1210).

9. The method as claimed in claim 8, wherein the plurality of PPG signals comprise a first PPG signal and a second PPG signal, and the at least one ECG lead signal comprises a first ECG lead signal, and the plurality of blood pressures comprise a first blood pressure and a second blood pressure, wherein the first blood pressure is estimated according to the first PPG signal and the first ECG lead signal, and the second blood pressure is estimated according to the second PPG signal and the first ECG lead signal, and the ABI is calculated according to the first blood pressure and the second blood pressure.

10. The method as claimed in claim 7, wherein the plurality of PPG signals and the plurality of skin voltages are sensed substantially simultaneously.

11. A computer readable storage medium having stored therein instructions, which when executed by a device, cause the device to:

sensing a plurality of photoplethysmogram (PPG) signals of a user;
sensing a plurality of skin voltages of the user;
estimating an ankle brachial pressure index (ABI) according to the plurality of PPG signals and the plurality of skin voltages,

wherein said plurality of PPG signals being provided by a plurality of sensors (110-1, 110-2), and said plurality of skin voltages being provided by a plurality of electrodes (120-1, 120-2),
**characterized by**
one of the plurality of PPG sensors (110-1, 110-2) and one of the plurality of electrodes (120-1, 120-2) are housed in a first sensor housing body (320-1), and another one of the plurality of PPG sensors (110-1, 110-2) and another one of the plurality of electrodes (120-1, 120-2) are housed in the second sensor housing body (320-2).

12. The computer readable storage medium as claimed in claim 11, wherein estimating the ABI comprises:

estimating a plurality of blood pressures according to the plurality of PPG signals and the at least one ECG lead signal; and
calculating the ABI according to the plurality of blood pressures.

13. The computer readable storage medium as claimed in claim 12, wherein the plurality of PPG signals comprise a first PPG signal and a second PPG signal, and the at least one ECG lead signal comprises a first ECG lead signal, and the plurality of blood pressures comprise a first blood pressure and a second blood pressure, wherein the first blood pressure is estimated according to the first PPG signal and the first ECG lead signal, and the second blood pressure is estimated according to the second PPG signal and the first ECG lead signal, and the ABI is calculated according to the first blood pressure and the second blood pressure.

FIG. 1

FIG. 2

FIG. 3

FIG. 4

FIG. 5

FIG. 6

FIG. 7

FIG. 8

FIG. 9

FIG. 10

FIG. 11

Start

Sensing a plurality of PPG signals of a user ⌐S1202

Sensing a plurality of skin voltages of the user ⌐S1204

Generating at least one ECG lead signal according to the plurality of skin voltages ⌐S1206

Estimating a plurality of blood pressures according to the plurality of PPG signals and the at least one ECG lead signal ⌐S1208

Estimating an ABI according to the plurality of blood pressures ⌐S1210

End

FIG. 12

Europäisches
Patentamt

European
Patent Office

Office européen
des brevets

**EUROPEAN SEARCH REPORT**

Application Number

EP 14 18 1718

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | X. J. A. Tan, K. P. Chua, J. Y. A. Foo: "Development of a Non-invasive System to Estimate Ankle Brachial Index" In: "The 15th International Conference on Biomedical Engineering - ICBME 2013, 4th to 7th December 2013, Singapore", 4 December 2013 (2013-12-04), Springer International Publishing, Switzerland, XP008172001, ISBN: 978-3-319-02913-9 vol. 43, pages 817-820, DOI: 10.1007/978-3-319-02913-9_210, | 1,4-15 | INV. A61B5/021 |
| Y | * page 818, column 1, paragraph 1 - page 819, column 1, paragraph 2 * ----- | 2,3 | |
| Y | US 2007/276632 A1 (BANET MATTHEW JOHN [US] ET AL) 29 November 2007 (2007-11-29) | 2,3 | |
| A | * paragraph [0016] * * paragraph [0034] - paragraph [0047] * ----- | 1,5,8,9, 13 | |
| A | EP 1 733 679 A2 (FUKUDA DENSHI KK [JP]) 20 December 2006 (2006-12-20) | 1,4-6, 8-10, 12-14 | |
| | * paragraph [0032] - paragraph [0034] * * paragraph [0040] * * paragraph [0044] * ----- | | |
| A | WO 2006/049571 A1 (LINDBERG LARS-GOERAN [SE]; JONSSON BJOERN [SE]) 11 May 2006 (2006-05-11) * paragraph [0012] * * paragraph [0050] * * paragraph [0070] * ----- | 1,4-6, 8-10, 12-14 | |

TECHNICAL FIELDS SEARCHED (IPC)

A61B

The present search report has been drawn up for all claims

| Place of search | Date of completion of the search | Examiner |
|---|---|---|
| The Hague | 12 September 2014 | Gooding Arango, J |

EPO FORM 1503 03.82 (P04C01)

## ANNEX TO THE EUROPEAN SEARCH REPORT
## ON EUROPEAN PATENT APPLICATION NO.

EP 14 18 1718

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2014

| Patent document cited in search report | | Publication date | Patent family member(s) | | Publication date |
|---|---|---|---|---|---|
| US 2007276632 | A1 | 29-11-2007 | NONE | | |
| EP 1733679 | A2 | 20-12-2006 | CN | 1879554 A | 20-12-2006 |
| | | | EP | 1733679 A2 | 20-12-2006 |
| | | | JP | 5179707 B2 | 10-04-2013 |
| | | | JP | 2006346288 A | 28-12-2006 |
| | | | KR | 20060132462 A | 21-12-2006 |
| | | | RU | 2334462 C2 | 27-09-2008 |
| | | | US | 2007004985 A1 | 04-01-2007 |
| WO 2006049571 | A1 | 11-05-2006 | EP | 2112902 A1 | 04-11-2009 |
| | | | US | 2008269619 A1 | 30-10-2008 |
| | | | WO | 2006049571 A1 | 11-05-2006 |

EPO FORM P0459

For more details about this annex : see Official Journal of the European Patent Office, No. 12/82